# EUROPEAN PATENT APPLICATION

(11) **EP 3 316 328 A1**
(43) Date of publication of application: **02.05.2018**
(21) Application number: 16814011.9
(22) Date of filing: 18.03.2016
(51) Int. Cl.: H01L 51/50, C07D 401/14, C09K 11/06

(54) **ORGANIC EL MATERIAL AND ORGANIC EL ELEMENT EMPLOYING SAME**

(30) Priority: 23.06.2015 JP 2015125942; 15.09.2015 JP 2015182242
(71) Applicant: Kaneka Corporation, Osaka-shi, Osaka 530-8288 (JP); Kyushu University National University Corporation, Fukuoka 812-8581 (JP)
(72) Inventor: HAYANO, Tetsuji, Osaka-shi Osaka 530-8288 (JP); ADACHI, Chihaya, Fukuoka-shi Fukuoka 812-8581 (JP)
(74) Representative: Hoffmann Eitle
(86) International application number: PCT/JP2016/058845
(87) International publication number: WO 2016/208240

(57) **Abstract**

An organic EL material according to the present invention includes a compound represented by the general formula (I). In the formula (I), R¹ and R² are each independently a hydrogen atom or any substituent, and are preferably each independently a substituent selected from the group consisting of a hydrogen atom, a cyano group, a halogen-substituted alkyl group, a halogen, a pyridyl group optionally having a substituent, and an electron-donating aromatic group. A is a substituent in which at least one heteroaryl group optionally having a substituent, or at least one aryl amino group optionally having a substituent is bonded to a carbon atom at the 4-position of the pyridine ring directly or through other aromatic group.

## Description

### TECHNICAL FIELD

The present invention relates to an organic EL material and an organic electroluminescence (EL) element using the same.

### BACKGROUND ART

An organic EL element includes at least one light-emitting layer between a pair of electrodes including an anode and a cathode. When a voltage is applied to the organic EL element, holes are injected into the light-emitting layer from the anode, and electrons are injected into the light-emitting layer from the cathode. The injected holes and electrons are recombined in the light-emitting layer.

When holes and electrons are recombined in the light-emitting layer, an organic molecule makes a state transition from a ground state (S₀ state) to an excited state. Here, according to the spin statistical law, 25% of the organic molecule is in a singlet lowest excited state (S₁ state), and 75% of the organic molecule is in a triplet lowest excited state (T₁ state). When the light-emitting layer includes two or more organic molecules such as a host and a dopant, organic molecule on which the recombination occurs varies depending on a combination of molecules. When recombination occurs on a molecule (*e.g.,* host) other than the dopant, energy is transferred from a material in an excited state to a dopant material, and the dopant material makes a state transition from the ground state to the excited state. Here, in principle, the abundance ratio between the S₁ state and the T₁ state follows a value in a host material. Specifically, 25% of the dopant material that has come into the excited state is in the S₁ state, and 75% of the dopant material is in the T₁ state. On the other hand, when recombination occurs on the dopant, the dopant material directly comes into the excited state, where 25% of the dopant material is in the S₁ state, and 75% of the dopant material is in the T₁ state.

A fluorescent material emits fluorescence in transition from the S₁ state to the S₀ state. Therefore, in principle, only 25% of the fluorescent material, which is in the S₁ state, can be made to contribute to light emission. On the other hand, phosphorescence is light emitted in transition from the T₁ state to the S₀ state. When intersystem crossing from the S₁ state to the T₁ state is efficiently performed in the phosphorescent dopant material, the internal quantum yield can be increased to 100% in principle. Thus, phosphorescent organic EL light emitting elements have been extensively developed, and new dopant materials and host materials have also been found.

In a light-emitting layer of a phosphorescent organic EL element, an iridium complex, a platinum complex or the like is used as a phosphorescent dopant material of the light-emitting layer. The host material is required to have a larger T₁-S₀ energy gap than that of the dopant material, and carbazole derivatives such as 4,4'-dicarbazolebiphenyl (CBP) are widely used.

On the other hand, CBP and CDBP have the problem that they have a large energy gap, and are therefore poor in ability to receive holes and electrons, leading to an increase in driving voltage of an organic EL element. In view of the above-mentioned problem, Patent Document 1 suggests that a bipolar compound in which a carbazolyl group as an electron-withdrawing part and a heteroarylene group as an electron-transporting part are bonded to each other is used as a phosphorescent host material. Patent Document 1 discloses that such a phosphorescent organic EL element is excellent in external quantum yield at a low driving voltage. Further, Patent Document 2 suggests that a bipolar compound in which a carbazolyl group and a cyano-substituted arylene group or a cyano-substituted heteroarylene group are bonded to each other is used as a phosphorescent host material.

As described above, an organic EL element using phosphorescence is capable of attaining high luminous efficiency. However, as described in Patent Documents 1 and 2, most of dopant materials that emit phosphorescence with high efficiency are metal complex compounds containing precious metals such as iridium and platinum, and thus have the problem that the materials are extremely expensive.

In recent years, as a technique for achieving an internal quantum yield of more than 25%, which is the theoretical upper limit for a fluorescent organic EL element while using an inexpensive fluorescent material, an organic EL element using thermally activated delayed fluorescence has been developed and attracted attention. In the thermally activated delayed fluorescent material, a difference ΔE_{ST} between the S₁ energy and the T₁ energy is small, and therefore a state transition (inverse intersystem crossing) from the T₁ state to the S₁ state is caused to occur by the thermal energy. When inverse intersystem crossing is caused to occur by thermal energy, fluorescence is emitted via the S₁ state from the T₁ state, so that an internal quantum yield of more than 25% can be achieved, and theoretically the internal quantum yield can be increased to 100%.

For example, Non-Patent Document 1 and Patent Document 3 indicate that a specific cyanobenzene derivative in which an electron-donating carbazolyl group is bonded to an electron-withdrawing dicyanobenzene is useful as a thermally activated delayed fluorescent material. Non-Patent Document 2 reports that an organic EL element using 1,2,3,5-tetrakis (9-carbazolyl) -4,6-dicyanobenzene (4 CzIPN) as a thermally activated delayed fluorescent material is essentially stable under electrical excitation, and has a durability lifetime comparable to that of a conventional phosphorescent organic EL element. Patent Document 4 indicates that 2,4-dicarbazolyl-3-cyanopyridine is useful as a thermally activated delayed fluorescent material. In addition, Patent Document 3 discloses a dicyanopyridine derivative having cyano groups at the 3- and 5-positions and aryl groups at the 2-, 4- and 6-positions as an example of an organic EL material using a cyanopyridine derivative.

### PRIOR ART DOCUMENTS

### PATENT DOCUMENTS

Patent Document 1: Japanese Patent Laid-open Publication No. 2010-241801
Patent Document 2: Japanese Patent Laid-open Publication No. 2009-094486
Patent Document 3: International Publication No. WO 2013/154064
Patent Document 4: International Publication No. WO 2014/129330

### NON-PATENT DOCUMENTS

Non-Patent Document 1: Nature, 492, 234 (2012)
Non-Patent Document 2: Scientific Reports, 3, 2127 (2013)
Non-Patent Document 3: J. Mater. Chem., 22, 8922 (2012)

### SUMMARY OF THE INVENTION

### PROBLEMS TO BE SOLVED BY THE INVENTION

A thermally activated delayed fluorescent material of thermally activated type attracts attention as an organic EL material with high efficiency and a long durability lifetime. As described above, the thermally activated delayed fluorescent material is required to have small ΔE_{ST}. In general, a compound having small ΔE_{ST} tends to have a small light-emission quantum yield. Non-Patent Document 1 describes that electron-withdrawing dicyanobenzene and an electron-donating carbazolyl group are twisted by steric hindrance to localize HOMO and LUMO, so that both of low ΔE_{ST} and a high quantum yield can be attained.

However, the types of thermally activated delayed fluorescent materials with a high quantum yield, which satisfy these characteristics are limited, and only some compounds have been confirmed to be useful. Further, a clear relationship has not been found yet between the backbone structures of an electron-withdrawing part and an electron-donating part or the type of a substituent group and the light-emission characteristics, and it is difficult to predict luminous efficiency and a light-emission wavelength based on a chemical structure. In view of these situations, an object of the present invention is to provide an organic EL material, particularly an organic EL material useful as a thermally activated delayed fluorescent material, and an organic EL element using the organic EL material.

### MEANS FOR SOLVING THE PROBLEMS

The present inventors have conducted studies, and resultantly found that a dicyanopyridine derivative having a cyano group as a substituent at the 3- and 5-positions of pyridine and a specific electron-donating substituent at the 4-position between these cyano groups is useful as an organic EL material capable of emitting thermally activated delayed fluorescence, leading to the present invention.

The present invention relates to an organic EL material including a compound represented by the following general formula (I).

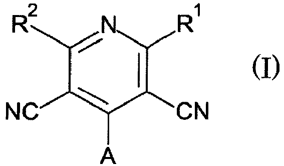

In the general formula (I), R¹ and R² are each independently a hydrogen atom or any substituent. A includes a heteroaryl group optionally having a substituent, or an arylamino group optionally having a substituent. The heteroaryl group or arylamino group in A is bonded directly to the carbon atom at the 4-position of the pyridine ring, or bonded to the carbon atom at the 4-position of the pyridine ring through another aromatic group.

Preferably, the organic EL material of the present invention is used as a light-emitting material in organic EL. In particular, when a difference ΔE_{ST} between S₁ energy and T₁ energy is 0.3 eV or less, the organic EL material can be used in a light-emitting layer of an organic EL element as an organic EL light-emitting material that emits delayed fluorescence, and the organic EL material is particularly useful as a light-emitting dopant material.

In the general formula (I), it is preferable that R¹ and R² are each independently a substituent selected from the group consisting of a hydrogen atom, a cyano group, a halogen-substituted alkyl group, a halogen, a pyridyl group optionally having a substituent, and an electron-donating aromatic group, and it is especially preferable that both R¹ and R² are hydrogen atoms.

In the above general formula (I), when the substituent A bonded to the carbon atom at the 4-position of the pyridine ring is a substituted aryl, the substituent of the substituted aryl is preferably a heteroaryl group or an arylamino group optionally having a substituent. Further, when A is a substituted aryl, and has a heteroaryl group as a substituent of the substituted aryl, A may further have a substituent different from the heteroaryl group at a position adjacent to a bonding position to the pyridine ring.

In the general formula (I), when the substituent A bonded to the carbon atom at the 4-position of the pyridine ring includes a heteroaryl group, the heteroaryl group is preferably a nitrogen-containing heteroaryl group, particularly preferably a carbazolyl group. Examples of the substituent A include substituents represented by the following formulae (II) and (III). In the formulae (II) and (III), Cz is a carbazolyl group optionally having a substituent.

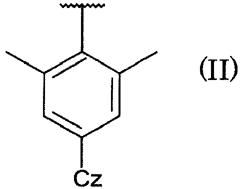

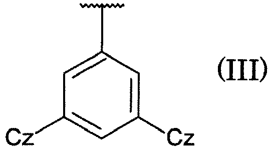

In the general formula (I), when the substituent A is a (substituted aryl) amino group with a nitrogen atom bonded to the carbon atom at the 4-position of the pyridine ring, the substituent of the substituted aryl is preferably a heteroaryl group. Examples of the substituent A include a substituent represented by the following formula (IV). In the formula (IV), Cz is a carbazolyl group optionally having a substituent.

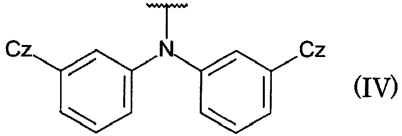

Further, the present invention relates to an organic EL element using the organic EL material. The organic EL element of the present invention includes a plurality of organic layers including a light-emitting layer between a pair of electrodes, and contains the organic EL material in at least one of the plurality of organic layers. The organic EL element of the present invention can be used in lighting fixtures, display devices, and so on.

### EFFECTS OF THE INVENTION

An organic EL material of the present invention can be used as a material of an organic layer of an organic EL element. In particular, when the organic EL material is used as a dopant material of a light-emitting layer, luminous efficiency can be improved by emission of delayed fluorescence.

### BRIEF DESCRIPTION OF THE DRAWINGS

FIG. 1 shows a schematic sectional configuration showing a configuration of an organic EL element according to an embodiment of the present invention.
FIG. 2A shows a light-emission spectrum of a compound 1 in a toluene solution.
FIG. 2B shows a light-emission spectrum of a compound 2 in a toluene solution.
FIG. 2C shows a light-emission spectrum of a compound 3 in a toluene solution.
FIG. 2D shows a light-emission spectrum of a compound 4 in a toluene solution.
FIG. 3A is a transient decay curve of fluorescence of compound 1 in a toluene solution.
FIG. 3B is a transient decay curve of fluorescence of compound 2 in a toluene solution.
FIG. 3C is a transient decay curve of fluorescence of compound 3 in a toluene solution.
FIG. 3D is a transient decay curve of fluorescence of compound 4 in a toluene solution.
FIG. 4 is a transient decay curve of fluorescence of an organic photoluminescence element of a thin-film of the compound 4.
FIG. 5 shows a light-emission spectrum of an organic photoluminescence element of a thin-film of the compound 4.
FIG. 6 is a transient decay curve of fluorescence of an organic photoluminescence element in a co-deposited film of a host material and the compound 4.
FIG. 7 shows a light-emission spectrum of an organic photoluminescence element in a co-deposited film of a host material and the compound 4.
FIG. 8 shows a light-emission spectrum of an organic photoluminescence element in a co-deposited film of a host material and the compound 4.
FIG. 9 shows a light-emission spectrum of an organic EL element.
FIG. 10 is a graph obtained by plotting a relationship between an applied voltage and a current density in an organic EL element.
FIG. 11 is a graph obtained by plotting a relationship between a current density and an external quantum yield in an organic EL element.

### MODE FOR CARRYING OUT THE INVENTION

### [Organic EL Material]

First, an organic EL material of the present invention will be described. The organic EL material of the present invention includes a compound represented by the following general formula (I).

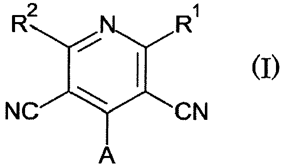

In the general formula (I), R¹ and R² are each independently a hydrogen atom or any substituent. A substituent A bonded to the carbon atom at the 4-position of the pyridine ring includes a heteroaryl group optionally having a substituent, or an arylamino group optionally having a substituent. Substituent A is electron-donating and the dicyanopyridine moiety is electron-withdrawing. In other words, the compound represented by the general formula (I) is a bipolar compound an electron-withdrawing dicyanopyridine part and the electron-donating substituent A are bonded to each other.

### <Substituent A>

### (Substituent A including heteroaryl)

The "heteroaryl group optionally having a substituent" in the substituent A is a group containing a substituted or unsubstituted aromatic heterocyclic ring. The aromatic heterocyclic ring is preferably one having 5 to 30 ring-forming atoms. Specific examples of the electron-donating aromatic heterocyclic ring include fused bicyclic rings such as indole, isoindole, thienoindole, indazole, purine, quinoline, and isoquinoline; fused tricyclic rings such as carbazole, acridine, β-carboline, acridone, perimidine, phenazine, phenanthridine, phenothiazine, phenoxazine, 1,7-phenanthroline, 1,8-phenanthroline, 1,9-phenanthroline, 1,10-phenanthroline, 2,7-phenanthroline, 2,8-phenanthroline, 2,9- phenanthroline, 3,7-phenanthroline and 3,8-phenanthroline; fused tetracyclic rings such as quindoline and quinindoline; and fused pentacyclic rings such as acrindoline. Among the above-mentioned aromatic heterocyclic rings, the aromatic heterocyclic ring contained in the substituent A is preferably a nitrogen-containing aromatic heterocyclic ring such as a carbazole ring, an indole ring, a thienoindole ring, an indoline ring, an acridine ring, or a phenoxazine ring, particularly preferably a carbazole ring.

The heteroaryl group optionally having a substituent may be bonded directly to the carbon atom at the 4-position of the pyridine ring, or bonded directly to the carbon atom at the 4-position of the pyridine ring through other substituent. When the heteroaryl group (hAr) is bonded directly to the carbon atom at the 4-position of the pyridine ring as shown in the following structural formula (101), it is preferable that a heteroatom (*e.g.,* nitrogen) of the heteroaryl group is bonded to the carbon atom at the 4-position of the pyridine ring.

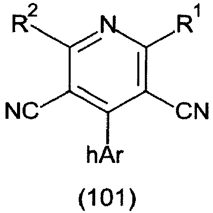

In the structural formula (101) and the following structural formulae, hAr is a heteroaryl group optionally having a substituent, and is preferably a substituted or unsubstituted carbazolyl group. When hAr is a substituted heteroaryl, an aromatic heterocyclic ring may be bonded as a substituent on the aromatic heterocyclic ring. When hAr has a plurality of aromatic heterocyclic rings, the plurality of aromatic heterocyclic rings may be the same or different.

When a nitrogen-containing fused polycyclic ring such as carbazole is bonded directly to the carbon atom at the 4-position of the pyridine ring, not only the substituent A has high electron-donating property, but also a bond between the pyridine ring and the substituent A (hAr) can be twisted by steric hindrance. When the bond is twisted, HOMO and LUMO are localized, and thus an energy difference ΔE_{ST} between a singlet lowest excited state (Si state) and a triplet lowest excited state (T₁ state) decreases, so that inverse intersystem crossing easily occurs by the thermal energy. The result of molecular orbital calculation of a system represented by the structural formula (101) shows that HOMO is localized in the substituent A, and LUMO is localized in the pyridine ring.

In the general formula (I), when a heteroaryl group is bonded to the carbon atom at the 4-position of the pyridine ring through other substituent, the "other substituent" is preferably an aromatic group, particularly preferably an aromatic group including a benzene ring. Examples of the compound in which a heteroaryl group is bonded to the carbon atom at the 4-position of the pyridine ring through a benzene ring include compounds having the structures described below.

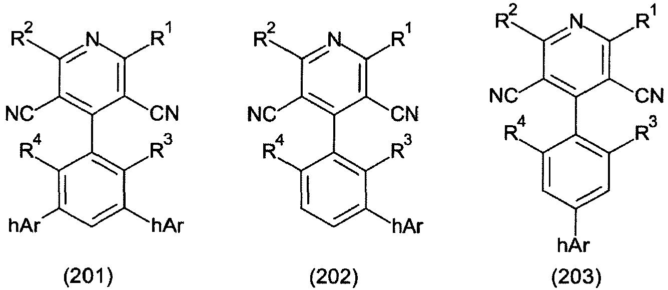

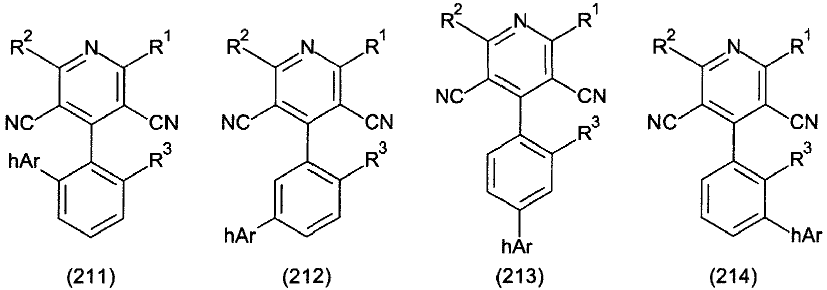

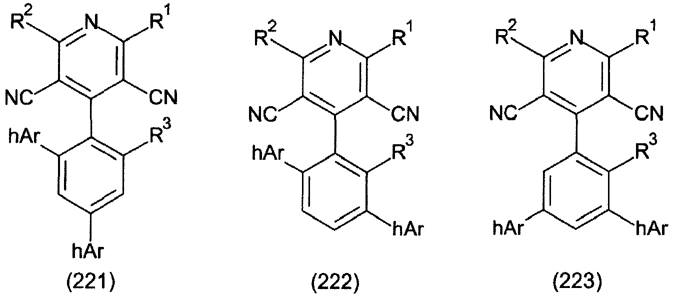

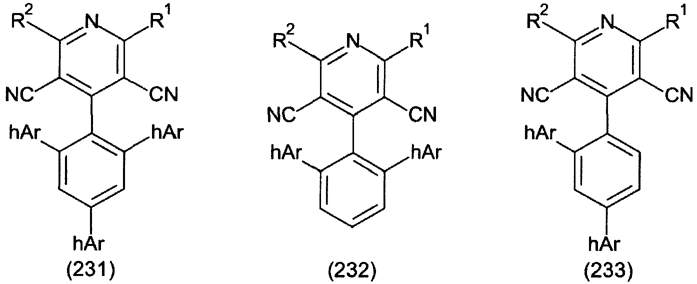

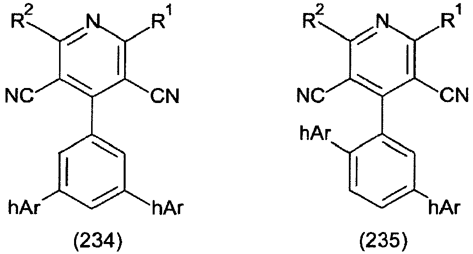

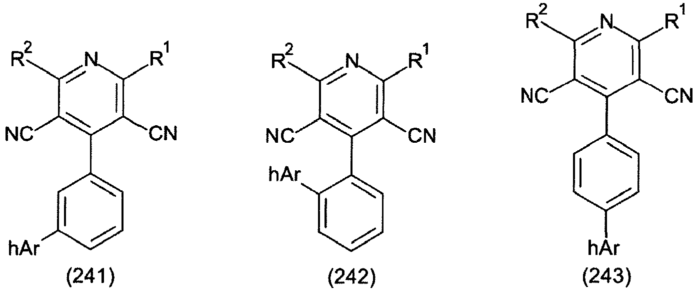

In the above structural formulae, R³ and R⁴ are each independently any substituent other than a hydrogen atom, and hAr is a heteroaryl group optionally having a substituent.

The compound of each of the structural formulae corresponds to a case where the substituent A in the general formula (I) is a substituted aryl, and a heteroaryl group is included as a substituent on the aryl. In the structural formulae (201), (202) and (203), among the above-mentioned structural formulae, the aromatic ring has substituents R³ and R⁴ each different from a heteroaryl group at positions adjacent to bonding positions to the carbon atom at the 4-position of the pyridine ring, *i.e*., at both o-positions of the benzene ring. In the structural formulae (211), (212), (213), (214), (221), (222) and (223), a substituent R³ different from the heteroaryl group is present at one of the o-positions of the benzene ring. Thus, when a substituent is present at a position adjacent to a bonding position to the carbon atom at the 4-position of the pyridine ring, a bond between the pyridine ring and the substituent A is easily twisted by steric hindrance between the substituents R³ and R⁴ and the cyano groups at the 3- and 5-positions of the pyridine ring.

When a heteroaryl group is present only at the p-position of the benzene ring as in the structural formula (243), a bond between the pyridine ring and the benzene ring is hardly twisted by steric hindrance of the heteroaryl group. On the other hand, the presence of a substituent at a position adjacent to a bonding position to the carbon atom at the 4-position of the pyridine ring as in the structural formulae (203) and (213) makes it possible to twist the bond.

Particularly, in the compound of the structural formula (203), the bond is easily twisted because substituents R³ and R⁴ are present at both of the positions adjacent to a bonding position to the carbon atom at the 4-position of the pyridine ring. In particular, when hAr is a substituted or unsubstituted carbazolyl group, the twist angle between dicyanopyridine as an acceptor part and a substituted aryl as a donor part is properly adjusted, and in addition, the electron density of both the parts are appropriately adjusted. Thus, luminous efficiency can be improved while ΔE_{ST} is reduced, so that an organic EL material useful as a delayed fluorescent material is obtained.

Specific examples of each of the substituents R³ and R⁴ include a halogen atom, a cyano group, a nitro group, a silyl group, an amino group, an alkyl group having 1 to 6 carbon atoms, a halogen-substituted alkyl group having 1 to 6 carbon atoms, a cycloalkyl group having 4 to 8 carbon atoms, a substituted or unsubstituted aryl group having 6 to 12 carbon atoms, an alkoxy group having 1 to 6 carbon atoms, a cycloalkoxy group having 4 to 8 carbon atoms, an aryloxy group having 6 to 12 carbon atoms, an alkylthio group having 1 to 6 carbon atoms, a cycloalkylthio group having 4 to 10 carbon atoms, an arylthio group having 6 to 12 carbon atoms, an alkoxycarbonyl group having 1 to 6 carbon atoms, an aryloxycarbonyl group having 6 to 12 carbon atoms, a sulfamoyl group having 1 to 6 carbon atoms, an acyl group having 1 to 6 carbon atoms, an acyloxy group having 1 to 6 carbon atoms, an amide group having 1 to 6 carbon atoms, a carbonyl group having 1 to 6 carbon atoms, a ureido group having 1 to 6 carbon atoms, a sulfinyl group having 1 to 6 carbon atoms, an alkylsulfonyl group having 1 to 6 carbon atoms, an aryl sulfonyl group having 6 to 12 carbon atoms, and an alkylamino group having 1 to 6 carbon atoms. Among them, a halogen atom, a cyano group, a methyl group, a trifluoromethyl group, a methoxy group, and a phenyl group are preferable, a methyl group and a trifluoromethyl group are especially preferable, and a methyl group is most preferable. When substituents R³ and R⁴ are present on the benzene ring, these substituents may be the same or different.

To summarize, among compounds in which a heteroaryl group is bonded to the carbon atom at the 4-position of the pyridine ring through a benzene ring, and a substituent is present at a position adjacent to a bonding position to the carbon atom at the 4-position of the pyridine ring, compounds in which the substituent A in the general formula (I) is a group represented by the following formula (II) are especially preferable. In the following formula (II) and the following structural formulae, Cz is a carbazolyl group optionally having a substituent.

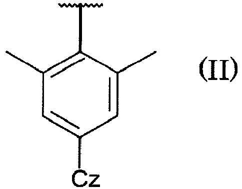

When the substituent A is a group represented by the formula (II), the twist angle of a bond between the acceptor part and the donor part is appropriately adjusted by steric hindrance of the cyano group of dicyanopyridine and the methyl group on the benzene ring. Thus, even when there is no substituent on the carbazole ring, a delayed fluorescent material having a short-wavelength light-emission peak and a high light-emission quantum yield is obtained.

When there is no substituent at the position adjacent to a bonding position to the carbon atom at the 4-position of the pyridine ring, the presence of at least one heteroaryl group at the o- or m-position of the benzene ring as in the structural formulae (231), (232), (233), (234), (235), (241) and (242) makes it possible to twist the bond by steric hindrance of the heteroaryl group.

Particularly, when a heteroaryl group optionally having a substituent is present at two m-positions of the benzene ring as shown in the structural formula (234), the twist angle of a bond between electron-withdrawing dicyanopyridine and the electron-donating substituent A with steric hindrance is appropriately adjusted, so that ΔE_{ST} tends to decrease. In particular, when hAr is a substituted or unsubstituted carbazolyl group, the twist angle between dicyanopyridine as an acceptor part and a substituted aryl as a donor part is properly adjusted, and in addition, the electron density of both the parts are appropriately adjusted. Thus, luminous efficiency can be improved while ΔE_{ST} is reduced, so that an organic EL material useful as a delayed fluorescent material is obtained. In the structural formula (234), the compound in which hAr is a carbazolyl group optionally having a substituent corresponds to a case where the substituent A in the general formula (I) is a group represented by the following formula (III).

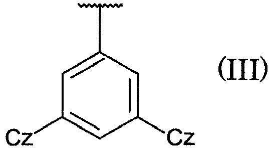

In the general formula (I), when a heteroaryl group is bonded to the carbon atom at the 4-position of the pyridine ring through other substituent, the "other substituent" is not limited to a group in which an aromatic ring is bonded directly to a pyridine ring. The "other substituent" may be, for example, a group in which an aromatic group is bonded to a pyridine ring through a nitrogen atom, and specific examples thereof include an arylamino group.

When a heteroaryl group is bonded to the carbon atom at the 4-position of the pyridine ring through an arylamino, the heteroaryl group is present as a substituent on the aromatic ring. The arylamino may be a monoarylamino or a diarylamino. The aryl amino is preferably a diarylamino, and is particularly preferably diphenylamino. Examples of the compound in which a heteroaryl group is bonded to the carbon atom at the 4-position of the pyridine ring through a diphenylamino group include compounds having the following structures.

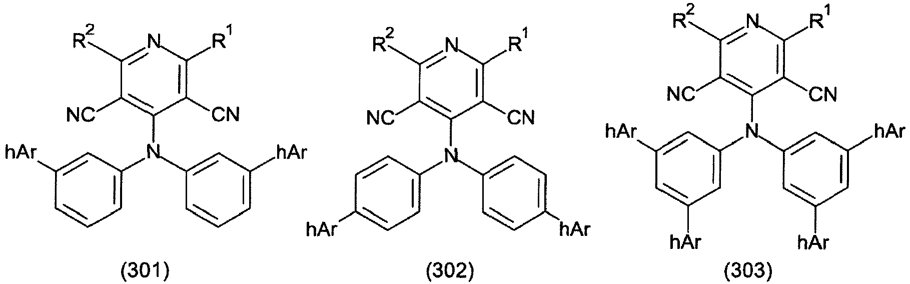

The structural formulae (301), (302) and (303) correspond to a case where in the general formula (I), A is a (substituted aryl) amino group in which a nitrogen atom is bonded to the carbon atom at the 4-position of the pyridine ring, and a heteroaryl group optionally having a substituent is present as a substituent of the substituted aryl. In particular, when a substituted or unsubstituted heteroaryl group is present at the m-position of a benzene ring bonded to a nitrogen atom as in the structural formula (301), a bond between the pyridine ring and the substituent A is easily twisted by steric hindrance, and the substituent A has high electron-donating property, so that ΔE_{ST} decreases, and thus inverse intersystem crossing by thermal energy easily occurs. In the structural formula (301), hAr is preferably a substituted or unsubstituted carbazolyl group. This compound corresponds to a case where the substituent A in the general formula (I) is a group of (IV) as described below.

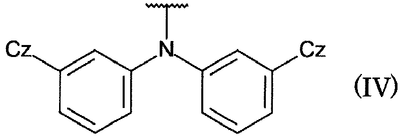

### (Substituent in Substituted Heteroaryl)

In the substituents or structural formulae shown above as an example, the heteroaryl group hAr has a substituent on a heterocyclic ring, so that a perturbation is added to ΔE_{ST} of the compound, or the light-emission wavelength when the compound is used in an organic EL material. The substituent on the heterocyclic ring of the heteroaryl may be either electron-donating or electron-withdrawing. For example, when a material having the same backbone at the electron-withdrawing part and having a light-emission wavelength on the shorter wavelength side is designed, it is preferable that the substituent on the heteroaryl is electron-withdrawing. Conversely, when a material having a light-emission wavelength on the longer wavelength side is designed, it is preferable that an electron-donating substituent is present on the heteroaryl.

The Hammett substituent constant σₚ of the electron-withdrawing substituent is preferably larger than 0, more preferably 0.1 or more, still more preferably 0.3 or more, especially preferably 0.6 or more. If the value of the Hammett substituent constant is positive, the substituent is electron-withdrawing, and the larger the value, the higher the electron withdrawing property. The Hammett substituent constant is described in detail in Hansch, C. et. al, Chem. Rev., 91, 165-195. (1991).

Specific examples of the electron-withdrawing substituent include a cyano group; a phenyl group; a nitro group; an acyl group; a formyl group; an acyloxy group; an acylthio group; an alkyloxycarbonyl group; an aryloxycarbonyl group; a halogen atom; an alkyl group substituted with at least two halogen atoms (preferably a perfluoroalkyl group substituted with two or more fluorine atoms, with the number of carbon atoms being preferably 1 to 6, more preferably 1 to 3; and specific examples thereof include a trifluoromethyl group); an alkoxy group substituted with at least two halogen atoms; an aryloxy group substituted with at least two halogen atoms; an alkylamino group substituted with at least two halogen atoms; an alkylthio group substituted with at least two halogen atoms; -COOR^{a} (R^{a} is a hydrogen atom or an alkyl group, preferably an alkyl group having 1 to 20 carbon atoms); -CONR^{b}₂ (R^{b}s are each independently a hydrogen atom, an alkyl group having 1 to 20 carbon atoms, an aryl group having 6 to 20 carbon atoms, or a heteroaryl group having 6 to 20 carbon atoms, preferably a hydrogen atom); SO₃M (M is a hydrogen atom or an alkali metal); groups including a phosphine oxide structure, such as a dialkylphosphono group, a diarylphosphono group, a dialkylphosphinyl group and a diarylphosphinyl group; a sulfonyl group; an alkylsulfonyl group; an arylsulfonyl group; an alkylsulfinyl group; an arylsulfinyl group; an acylthio group; a sulfamoyl group; a thiocyanate group; a thiocarbonyl group; an imino group; an imino group substituted with a N atom; a carboxy group or a salt thereof; an acylamino group; an azo group; a selenocyanate group; a heteroaryl group; an aryl group having a substituent of an electron-withdrawing substituent with σₚ of more than 0 (preferably with σₚ of more than 0.3).

Among the above-mentioned electron-withdrawing substituents, one selected from the group consisting of a cyano group, a perfluoroalkyl group (particularly a trifluoromethyl group) and a heteroaryl group is preferable. Preferably, the heteroaryl group has a heterocyclic ring structure containing a nitrogen atom or a sulfur atom as a heteroatom. Specific examples of the electron-withdrawing heteroaryl group include an oxadiazolyl group, a benzothiadiazolyl group, a tetrazolyl group, a thiazolyl group, an imidazolyl group and a pyridyl group. In the heteroaryl group, one of a carbon atom or a heteroatom may be bonded to an aromatic heterocyclic ring. For improving electron-withdrawing property, it is preferable that a carbon atom is bonded to an aromatic heterocyclic ring. Among the above-mentioned heteroaryl groups, one selected from the group consisting of a 2-pyridyl group, a 3-pyridyl group and a 4-pyridyl group is especially preferred.

In particular, when the electron-withdrawing group is a cyano group, the light-emission wavelength tends to be shortened. For example, when the substituent A is a group represented by the formula (III) or formula (IV), a compound in which the substituent on the carbazole ring is a cyano group is useful as a blue delayed fluorescent material. When the electron-withdrawing group is a pyridyl group, particularly when the electron-withdrawing group is a 4-pyridyl group, the light-emission quantum yield tends to be increased. The position of the electron-withdrawing group on the carbazole ring is not particularly limited. When an electron-withdrawing substituent is present at the 3-position and/or 6-position, the light-emission wavelength tends to be shortened, or the light-emission quantum yield tends to be improved.

When the light-emission wavelength is shortened, it is preferable that the heteroaryl group does not have a substituent other than an electron-withdrawing substituent (*i.e*., the heteroaryl group does not have an electron-donating substituent). When the heteroaryl group is a carbazolyl group having a substituent, it is preferable that the substituted carbazolyl group Cz has one of the following structures for shortening the light-emission wavelength. In the following (Cz21) and (Cz22), Py is a 2-pyridyl group, a 3-pyridyl group or a 4-pyridyl group, especially preferably a 4-pyridyl group. Two Pys in the following (Cz22) may be the same or different. They are preferably the same.

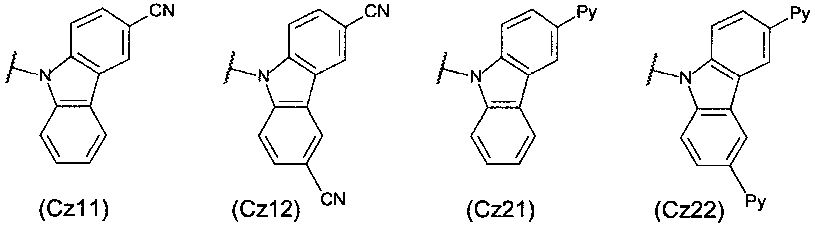

When the light-emission wavelength is lengthened, it is preferable that an electron-donating substituent is present on the heteroaryl. Examples of the electron-donating substituent include an alkyl group, an alkoxy group, a substituted or unsubstituted aryl group, and a substituted or unsubstituted heteroaryl group. As described above, the pyridyl group is electron-withdrawing, but when a 2-pyridyl group is bonded on a carbazole ring, the light-emission wavelength tends to be lengthened.

A compound in which the substituent A is a group represented by the general formula (II) (*e.g.,* a compound of the structural formula (203)) tends to emit light having a short wavelength, and therefore an electron-donating substituent may be introduced into the heteroaryl group to lengthen the light-emission wavelength. When the heteroaryl group is a carbazolyl group having a substituent, it is preferable that the substituted carbazolyl group Cz has one of the following structures for lengthening the light-emission wavelength.

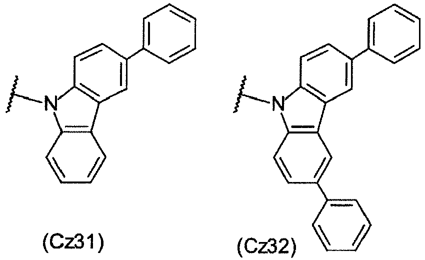

In particular, when the substituted carbazolyl group Cz is substituted with a phenyl group at the 3- and 6-positions on the carbazole ring (Cz32 above), not only the light-emission wavelength tends to be lengthened, but also ΔE_{ST} tends to decrease, so that a delayed fluorescent material with extremely high luminous efficiency is obtained. In addition, the presence of a plurality of substituents on the carbazole ring increases the molecular weight, so that heat resistance tends to be improved when the compound is used in an organic EL element.

### (Sub stituent A Containing Arylamino)

The "arylamino group optionally having a substituent" in the substituent A is a group in which at least one aryl group is bonded to a nitrogen atom. The substituent A containing an arylamino group may contain a heteroaryl group, or is not required to contain a heteroaryl group. The arylamino group of the substituent A may be bonded to the carbon atom at the 4-position of the pyridine ring through other aromatic group.

Examples of the compound in which the substituent A contains an arylamino group, and the nitrogen atom of the arylamino group is bonded directly to the carbon atom at the 4-position of the pyridine ring, among compounds of the general formula (I), include compounds in which a heteroaryl group is bonded to the carbon atom at the 4-position of the pyridine ring through an arylamino as in the structures (301), (302) and (303).

Examples of the compound in which the substituent A contains an arylamino group, and the nitrogen atom of the arylamino group is bonded to the carbon atom at the 4-position of the pyridine ring through other aromatic group, among compounds of the general formula (I), include compounds in which an arylamino group is bonded at the p-position of a benzene ring bonded to the carbon atom at the 4-position of the pyridine ring.

The arylamino may be a monoarylamino or a diarylamino. The aryl amino is preferably a diarylamino, particularly preferably diphenylamino. In particular, when a substituent is present at a position adjacent to a bonding position to the carbon atom at the 4-position of the pyridine ring, a bond between the pyridine ring and the substituent A is easily twisted by steric hindrance between the substituent and the cyano groups at the 3- and 5-positions of the pyridine ring, so that ΔE_{ST} of the compound tends to decrease.

Examples of the compound having a substituent at a position adjacent to a bonding position to the carbon atom at the 4-position of the pyridine ring include compounds of the following structural formulae (401) and (402). Specific examples of the substituents R³ and R⁴ in the following structural formulae (401) and (402) are the same as the specific examples of the substituents R³ and R⁴ described above.

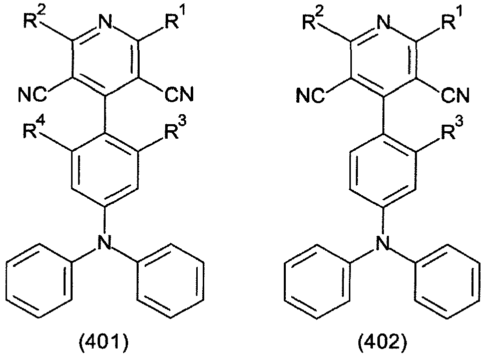

### <R¹ and R2>

In the above general formula (I), a hydrogen atom or any substituent is bonded to the carbon atoms at the 2- and 6-positions of the pyridine ring as R¹ and R², respectively.

When substituents R¹ and R² on the pyridine ring, and a substituent on the aryl or heteroaryl of the substituent A are present in the compound represented by the general formula (I), a perturbation is added to the twist angle of a bond between dicyanopyridine as an acceptor part and the substituent A as a donor part, and the electron densities of both the parts. Due to the presence of substituents R¹ and R², it is possible to optimize the light-emission wavelength and the light-emission quantum yield by finely adjusting the electron density of the dicyanopyridine part as an acceptor part.

When each of R¹ and R² is a substituent other than a hydrogen atom, the substituent may be electron-withdrawing or electron-donating. Examples of the electron-withdrawing substituent include a cyano group, a halogen-substituted alkyl group, a halogen, a pyridyl group optionally having a substituent. Examples of the electron-donating substituent include an electron-donating aromatic group. Specific examples of the electron-donating aromatic group include a substituted or unsubstituted heteroaryl group containing an electron-donating aromatic heterocyclic ring as described above.

The compound represented by the general formula (I) is a bipolar compound an electron-withdrawing dicyanopyridine part and the electron-donating substituent A are bonded to each other, and as described above, by twisting the bond between the pyridine ring and the substituent A, HOMO and LUMO can be localized in the electron-donating part and the electron-withdrawing part, respectively, to reduce ΔE_{ST}. On the other hand, for achieving a high light-emission quantum yield, it is necessary to increase the overlap of the orbits of the localized HOMO and LUMO to increase the emission rate constant. By attaining both of these conflicting characteristics, a compound having small ΔE_{ST} and a high light-emission quantum yield is obtained. The overlap of HOMO and LUMO can be optimized by adjusting the twist and the electron density of each part. In the compound represented by the general formula (I), it is preferable that both the substituents R¹ and R² of the acceptor part are hydrogen from the viewpoints of a light-emission wavelength and a light-emission quantum yield.

### <Characteristics of Organic EL Material>

The compound represented by the general formula (I) is useful as an organic EL material, and particularly useful as a light-emitting material to be used in a light-emitting layer of an organic EL element. In particular, a compound having a small difference ΔE_{ST} between S₁ energy and T₁ energy has a high probability of occurrence of inverse intersystem crossing from the T₁ state to the S₁ state with thermal energy, and is thus useful as a light-emitting material that emits delayed fluorescence. In the compound represented by the general formula (I), the difference ΔE_{ST} between S₁ energy and T₁ energy is preferably 0.3 eV or less, more preferably 0.24 eV or less for using the compound as a delayed fluorescent material.

When the organic EL material of the present invention is used as a light-emitting material of an organic EL element, injection of carriers (holes and electrons) into the light-emitting material from an anode and a cathode causes the light-emitting material to make a transition into an excited state due to carrier recombination, and light is emitted at the time when excitons make a transition into a ground state. According to the spin statistical law, 25% of the excitons are in a singlet excited state (Si) and 75% of the excitons are in a triplet excited state (T₁).

In the delayed fluorescent material, transition from a triplet excited state to a singlet excited state (inverse intersystem crossing) occurs due to triplet-triplet annihilation or absorption of thermal energy, and fluorescence is emitted in transition from the singlet excited state to the ground state. Fluorescence generated via inverse intersystem crossing in this way is delayed fluorescence. In the organic EL element, a "thermally activated delayed fluorescent material" in which inverse intersystem crossing occurs due to absorption of thermal energy is particularly useful.

When a delayed fluorescent material is used in the organic EL element, excitons in a singlet excited state emit fluorescence as usual. On the other hand, excitons in a triplet excited state absorb thermal energy generated by the device, and is excited to a singlet excited state (subjected to inverse intersystem crossing) to emit fluorescence. Since fluorescence generated via inverse intersystem crossing is light emitted from singlet excitation, the fluorescence is light having a wavelength identical to that of light emitted by excitons excited directly to a singlet excited state from a ground state. However, the lifetime (light-emission lifetime) of fluorescence generated via inverse intersystem crossing is longer than that of usual fluorescence or phosphorescence, and is therefore observed as fluorescence that is delayed as compared to the usual fluorescence or phosphorescence. This fluorescence can be defined as delayed fluorescence.

When such a thermally activated exciton transfer mechanism is used, the ratio of a singlet excited state, which is usually produced in a ratio of only 25%, can be increased to more than 25% by absorbing thermal energy after injection of carriers. In particular, when a compound that emits intense fluorescence and delayed fluorescence even at low temperatures of lower than 100°C is used, inverse intersystem crossing from a triplet excited state to a singlet excited state is caused to occur sufficiently by heat from the device, so that delayed fluorescence is emitted, and therefore luminous efficiency can be dramatically improved.

### [Organic EL Element]

### <Configuration of Organic EL Element>

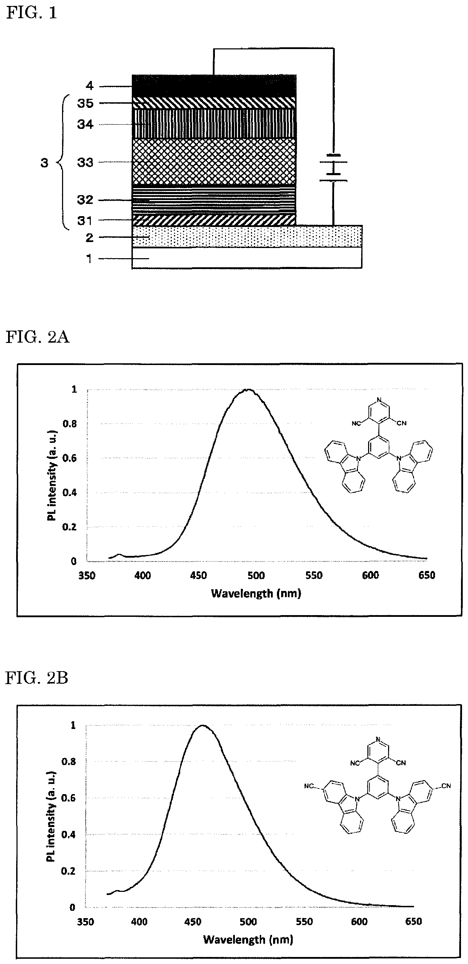

The organic EL element includes a plurality of organic layers between a pair of electrodes, and at least one of the organic layers is a light-emitting layer. FIG. 1 is a schematic sectional view showing a configuration of the organic EL element according to one embodiment. This element includes an anode 2 and a cathode 4 on a substrate 1, and an organic layer 3 between the pair of electrodes. The organic layer 3 has at least one light-emitting layer. The organic EL element of the present invention may have a light-emitting layer between a pair of electrodes, and is not limited to the configuration shown in FIG. 1. Hereinafter, each member and each layer of the organic EL element will be described.

### (Substrate)

Preferably, the organic EL element has a pair of electrodes 2 and 4 and the organic layer 3 on the substrate 1. The material of the substrate is not particularly limited, and is appropriately selected from, for example, a transparent substrate such as glass, a silicon substrate, a flexible film substrate and the like. In the case of an organic EL element of bottom emission type in which light is extracted from the substrate side, the substrate has a transmittance of preferably 80% or more, more preferably 90% or more, in a visible light range, for improving light extraction efficiency.

### (Anode)

Although the material of the anode is not particularly limited, a metal having a large work function (*e.g.,* 4 eV or more), an alloy, a metal oxide, electrically conductive compound, or a mixture thereof is preferably used. Specific examples of the material of the anode include thin-films of metals such as Au, and metal oxides such as indium tin oxide (ITO), indium zinc oxide (IZO), zinc oxide and tin oxide. Among them, ITO or IZO which is metal oxide having high transparency is preferably used for improving extraction efficiency of light generated from the light-emitting layer and facilitating patterning. The metal oxide that forms the anode may contain a dopant such as aluminum, gallium, silicon, boron or niobium as necessary.

### (Cathode)

Although the material of the cathode is not particularly limited, a metal having a small work function (*e.g.,* 4 eV or less), an alloy, a metal oxide, electrically conductive compound, or a mixture thereof is preferably used. Examples of the metal having a small work function include Li for alkali metals, and Mg and Ca for alkaline earth metals. In addition, a metal alone composed of a rare earth metal or the like, or an alloy of such a metal and Al, In, Ag or the like can also be used. Further, as disclosed in Japanese Patent Laid-open Publication No. 2001-102175, etc., a metal complex compound containing at least one selected from the group consisting of an alkaline earth metal ion and an alkali metal ion can also be used in an organic layer that is in contact with the cathode. In this case, it is preferable to use, as the cathode, a metal capable of reducing a metal ion in the complex compound to a metal in vacuum, such as Al, Zr, Ti, Si or the like, or an alloy containing such metals.

For extracting light from the light-emitting layer, it is preferable that one of the anode and the cathode is light-transmissive, and specifically, the transmittance in the visible light range is preferably 70% or more, more preferably 80 %, still more preferably 90% or more. When both the anode and the cathode are light-transmissive, an organic EL element capable of extracting light from both the anode side and the cathode side can be prepared.

### (Organic Layer)

The organic layer 3 may include organic layers such as a hole transport layer, a hole injection layer, an electron blocking layer, a hole blocking layer, an electron injection layer, an electron transport layer and an exciton blocking layer in addition to a light-emitting layer 33. The hole transport layer may be a hole injection/transport layer having a hole injection function, and the electron transport layer may be an electron injection/transport layer having an electron injection function. In the mode shown in FIG. 1, the organic layer 3 has a hole injection layer 31 and a hole transport layer 32 on the anode 2 side of the light-emitting layer 33, and an electron transport layer 34 and electron injection Layer 35 on the cathode 4 side of the light-emitting layer 33. In the organic EL element of the present invention, at least one of these organic layers contains one or more organic EL materials including a compound represented by the general formula (I).

### (Light-Emitting Layer)

The light-emitting layer is a layer that emits light after excitons are generated by recombination of holes and electrons injected from the anode and the cathode, respectively. In the organic EL element of the present invention, it is preferable that the light-emitting layer contains one or more organic EL materials including a compound represented by the general formula (I). The organic EL element may be one in which the organic EL material is used alone in the light-emitting layer. It is preferable the organic EL element is one in which the light-emitting layer contains a dopant material and a host material, and the dopant material includes the above-mentioned organic EL material. When the light-emitting layer contains a dopant material and a host material, singlet excitons and triplet excitons generated in the organic EL material (light-emitting dopant material) can be confined in the light-emitting layer, so that luminous efficiency tends to be improved.

When the organic EL material in the present invention is included as a dopant material of the light-emitting layer, light is emitted from the dopant material. The emission of light may include both of emission of fluorescence and emission of delayed fluorescence. In particular, when ΔE_{ST} of the organic EL material is 0.3 eV or less, delayed fluorescence tends to be easily emitted. ΔE_{ST} of an organic EL material used as a thermally activated delayed fluorescent material is preferably 0.24 eV or less.

A part of light emitted from the light-emitting layer may be light emitted from the host material. When the light-emitting layer contains a host material and a dopant material, the content of the dopant material in the light-emitting layer is preferably 0.1 to 49% by weight, more preferably 0.5 to 40% by weight, still more preferably 1 to 30% by weight. The content of the host material in the light-emitting layer is preferably 51 to 99.9% by weight, more preferably 60 to 99.5% by weight, still more preferably 70 to 99% by weight.

The host material is preferably a compound which exhibits favorable film-forming property, and can ensure favorable dispersibility of the dopant material. Further, it is preferable that in the host material, at least one of singlet excitation energy and triplet excitation energy has a higher value as compared to the dopant material. When the excitation energy of the host material is higher than the excitation energy of the dopant material, singlet excitons and triplet excitons generated in the dopant can be confined in the light-emitting layer, so that light emission efficiency can be improved.

For improving luminous efficiency, it is preferable that energy transfer from the singlet excitons of the host material to the dopant material actively occurs. Therefore, the singlet excitation energy of the host material is preferably higher than the singlet excitation energy of the dopant material. For increasing the intermolecular energy transition probability between the host material and the dopant material, a difference in singlet excitation energy between the host material and the dopant material is preferably 1 eV or less, more preferably 0.5 eV or less.

It is preferable that the host material has both hole transport performance and electron transport performance, and it is preferable that a difference between hole transport property and electron transport property is small. Specifically, the ratio of a hole mobility and an electron mobility, which is an index of transport performance, is preferably in a range of 0.002 to 500.

Specific examples of the host material include carbazole-based compounds, arylsilane-based compounds, phosphorus oxide-based compounds, oxadiazole-based compounds and quinolinol-based metal complexes. Examples of the carbazole-based compound include N, N'-dicarbazolyl-4,4'-biphenyl (CBP) and N, N-dicarbazolyl-3,5-benzene (mCP). Examples of the arylsilane-based compound include p-bis(triphenylsilyl)benzene (UGH2). Examples of the phosphorus oxide-based compound include 4,4'-bis(diphenylphosphoryl)-1,1'-biphenyl (PO1) and bis(2-(diphenylphosphino)phenyl)ether oxide (DPEPO). For the host material, one material may be used alone, or two or more materials may be used in combination.

### (Hole Transport Layer and Hole Injection Layer)

Preferably, the organic layer 3 has the hole injection layer 31 and the hole transport layer 32 on the anode 2 side of the light-emitting layer 33. The hole transport material has any of hole injection and transport properties and electron barrier property, and may be either an organic material or an inorganic material.

The hole transport material is preferably a compound which is easily radically cationized, and examples thereof include aryl amine-based compounds, imidazole-based compounds, oxadiazole-based compounds, oxazole-based compounds, triazole-based compounds, chalcone-based compounds, styrylanthracene-based compounds, stilbene-based compounds, tetraarylethene-based compounds, triarylamine-based compounds, triarylethene-based compounds, triarylmethane-based compounds, phthalocyanine-based compounds, fluorenone-based compounds, hydrazine-based compounds, carbazole-based compounds, N-vinylcarbazole-based compounds, pyrazoline-based compounds, pyrazolone-based compounds, phenylanthracene-based compounds, phenylenediamine-based compounds, polyarylalkane-based compounds, polysilane-based compounds and polyphenylenevinylene-based compounds.

In particular, the arylamine compound is easily radically cationized, and also has a high hole mobility, and thus the arylamine compound is suitable as a hole transport material. Among hole transport materials containing an arylamine compound, triarylamine derivatives such as 4,4'-bis [N-(2-naphthyl)-N-phenyl-amino]biphenyl (α-NPD) are preferable.

### (Electron Transport Layer and Electron Injection Layer)

Preferably, the organic layer 3 has the electron injection layer 35 and the electron transport layer 34 on the cathode 4 side of the light-emitting layer 33. The electron transport material has any of electron injection and transport properties and hole barrier property, and may be either an organic material or an inorganic material.

The electron transport material is preferably a compound which is easily radically anionized, and examples thereof include nitro-substituted fluorene derivatives, diphenylquinone derivatives, thiopyran dioxide derivatives, carbodiimides, fluorenylidenemethane derivatives, anthraquinodimethane and anthrone derivatives, oxadiazole derivatives, thiadiazole derivatives, phenanthroline derivatives, quinoline derivatives and quinoxaline derivatives. Specific examples of the electron transport material include 2,9-dimethyl-4,7-diphenyl-1,10-phenanthroline (BCP) and tris[(8-hydroxyquinolinate)]aluminum (III) (Alq₃). Among them, Alq₃ is preferably used from the viewpoint of versatility.

### (Blocking Layer)

A blocking layer may be provided for the purpose of preventing diffusion of holes, electrons or excitons present in the light-emitting layer out of the light emitting layer. The electron blocking layer is disposed between the light-emitting layer and the hole transport layer, and prevents diffusion of electrons to the hole transport layer side through the light-emitting layer. The hole blocking layer is disposed between the light-emitting layer and the electron transport layer, and prevents diffusion of holes to the electron transport layer side through the light-emitting layer. For the hole blocking layer, the same material as that of the electron transport layer can be used. For the electron blocking layer, the same material as that of the hole transport layer can be used.

The exciton blocking layer is a layer for preventing diffusion of excitons, which are generated by recombination of holes and electrons in the light-emitting layer, to the charge transport layer and the hole transport layer. By providing the exciton blocking layer, excitons can be efficiently confined in the light-emitting layer, so that the luminous efficiency of the element can be improved. The exciton blocking layer can be disposed on either the anode side or the cathode side of the light-emitting layer, and may be disposed on both the sides. For the blocking layer, a material is preferably used in which at least one of singlet excitation energy and triplet excitation energy is higher than singlet excitation energy and triplet excitation energy of the light-emitting dopant material.

### <Preparation of Organic EL Element>

The method for forming an electrode and an organic layer is not particularly limited, and a dry process such as a sputtering method, a CVD method or a vacuum vapor deposition method, or a wet process such as a spin coating method, or any of various printing methods is appropriately employed. The light-emitting layer containing a host material and a dopant material can be formed by, for example, co-evaporating the host material and the dopant material. Here, the host material and the dopant material may be mixed beforehand.

The organic EL element of the present invention may be one in which a compound represented by the general formula (I) is used as a material of an organic layer other than the light-emitting layer. When a compound represented by the general formula (I) is used in a layer other than the light-emitting layer, compounds to be used in the light-emitting layer and in the layer other than the light-emitting layer may be the same or different.

In practical use, it is preferable that a part or the whole of the organic EL element is sealed with sealing glass or a metal cap under an inert gas atmosphere, or covered with a protecting layer of an ultraviolet-curable resin or the like for minimizing degradation in a use environment.

The organic EL element of the present invention emits light when an electric field is applied between the anode and the cathode. Here, when the emission of light is caused by singlet excitation energy, light having a wavelength corresponding to the level of the energy is observed as a fluorescence emission and a delayed fluorescence emission. When the emission of light is caused by triplet excitation energy, light having a wavelength corresponding to the level of the energy is observed as phosphorescence. Since usual fluorescence has a fluorescence lifetime shorter than a delayed fluorescence emission, the light-emission lifetime can be distinguished by fluorescence and delayed fluorescence.

In the organic EL element including an organic EL material of the present invention as a dopant material of the light-emitting layer, it is preferable that the dopant material causes inverse intersystem crossing by thermal energy as described above. Excitons caused to make a transition into a singlet excited state by inverse intersystem crossing emits thermally activated delayed fluorescence. Thus, the organic EL element of the present invention exhibits a high internal quantum yield, and can serve as an energy-saving light source with low power consumption.

The organic EL element of the present invention can be effectively applied to lighting fixtures, display devices, and the like. Examples of the display device include liquid crystal display devices using an organic EL element as a lighting device (backlight), and organic EL display devices using an organic EL element as a display panel. Details of the organic EL display device can be found in "Organic EL Display", written by Shizuo TOKITO, Chihaya ADACHI and Hideyuki MURATA (Ohmsha, Ltd.), etc.

### EXAMPLE

The present invention will be more specifically described below by showing synthesis examples, and evaluation results of compounds obtained in the synthesis examples, but the present invention is not limited to the Examples below.

### [Synthesis Example 1]

In Synthesis Example 1, compound 1 was synthesized in accordance with the following scheme.

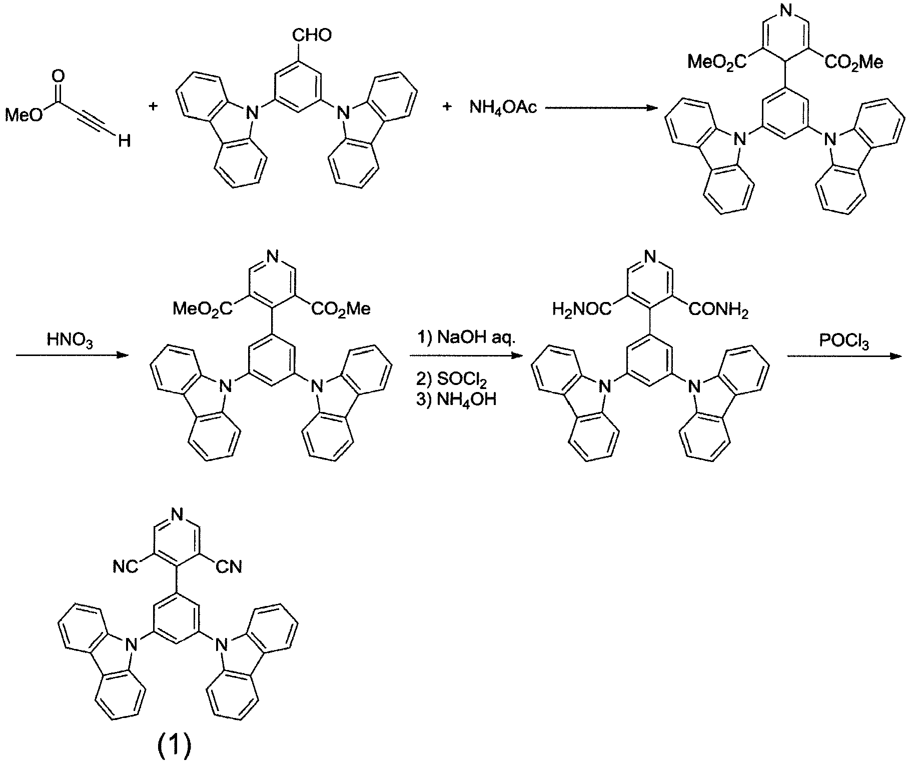

An acetic acid (30 ml) solution containing 3,5-(dicarbazolyl)-benzaldehyde (11.94 g), methyl propiolate (5.4 ml) and ammonium acetate (2.7 g) was heated and refluxed for 5 hours. The solution was cooled to room temperature, and the reaction solution was then poured into water. The desired product was extracted with chloroform, dried over magnesium sulfate, and then concentrated under reduced pressure. The resulting mixture was purified by silica gel chromatography to obtain 15.48 g of 1,4-dihydropyridine compound (yield: 94%).

A 20% nitric acid aqueous solution (10 ml) was added to the 1,4-dihydropyridine compound (0.5 g) obtained as described above, and the mixture was stirred under an oil bath heating condition at 70°C for 1 hour. After being cooled to room temperature, the reaction solution was poured into a sodium hydrogen carbonate aqueous solution to be neutralized, and the solid was collected by filtration. The resulting mixture was purified by silica gel chromatography to obtain 0.225 g of dimethyl-4-((3,5-dicarbazolyl)phenyl)pyridine-3,5-dicarboxylate (yield: 45%).

THF (90 ml), methanol (30 ml) and a 2N sodium hydroxide aqueous solution (40 ml) were added to dimethyl 4-((3,5-dicarbazolyl)phenyl)pyridine-3,5-dicarboxylate (5.45 g), and the mixture was heated and refluxed for 1.5 hours. After the reaction solution was cooled to room temperature, 3N hydrochloric acid was added dropwise to the reaction solution to adjust pH to 4. The resulting solid was collected by filtration, and dried under reduced pressure to obtain 4.42 g of 4-((3,5-dicarbazolyl) phenyl) pyridine-3,5-dicarboxylic acid (yield: 85%).

Thionyl chloride (50 ml) and a few drops of DMF were added to the 4-((3,5-dicarbazolyl)phenyl)pyridine-3,5-dicarboxylic acid (3.52 g) obtained as described above, and the mixture was heated and refluxed for 3.5 hours. The thionyl chloride was distilled off under reduced pressure, a small amount of dichloromethane was then added to dissolve the residue, and the solution was added dropwise to aqueous ammonia (100 ml). The resulting solid was collected by filtration, and dried under reduced pressure to obtain 3.31 g of 4-((3,5-dicarbazolyl)phenyl)pyridine-3,5-dicarboxamide (yield: 94%).

Phosphorus oxychloride (30 ml) was added to 4-((3,5-dicarbazolyl)phenyl)pyridine-3,5-dicarboxamide (3.86 g), and heated and refluxed for 2 hours. After being returned to room temperature, the reaction solution was poured into a beaker containing sodium hydroxide and ice. The resulting solid was collected by filtration, and then heated and refluxed in 2-propanol (300 ml) for 1 hour. After the solution was returned to room temperature, the solid was collected by filtration, and dried under reduced pressure to obtain 2.07 g of a desired product (yield: 57%).

The resulting compound 1 was further subjected to sublimation purification to obtain a sample for evaluation. It was confirmed by ¹H-NMR that the resulting compound was the compound 1. The measurement results of ¹H-NMR were as follows: ¹H-NMR (500 MHz, CDCl₃); δ = 9.20 (s, 2H), 8.16 (m, 5H), 7.86 (d, 2H), 7.73 (d, 4H), 7.49 (t, 4H), 7.33 (t, 4H).

From the results of analysis of a thin-film by atmospheric photoelectron spectroscopy (AC-3 manufactured by RIKEN KEIKI Co., Ltd.), the HOMO of the compound 1 was estimated to be 6.35 eV. From the long wavelength absorption edge of an absorption spectrum of the compound 1, the HOMO-LUMO band gap was estimated to be 3.60 eV, and the LUMO was estimated to be 2.75 eV.

### [Synthesis Example 2]

In Synthesis Example 2, except that 3,5-di(3-cyanocarbazolyl)-benzaldehyde was used in place of 3,5-(dicarbazolyl)-benzaldehyde, the same procedure as in Synthesis Example 1 was carried out to synthesize a compound 2 in accordance with the following scheme.

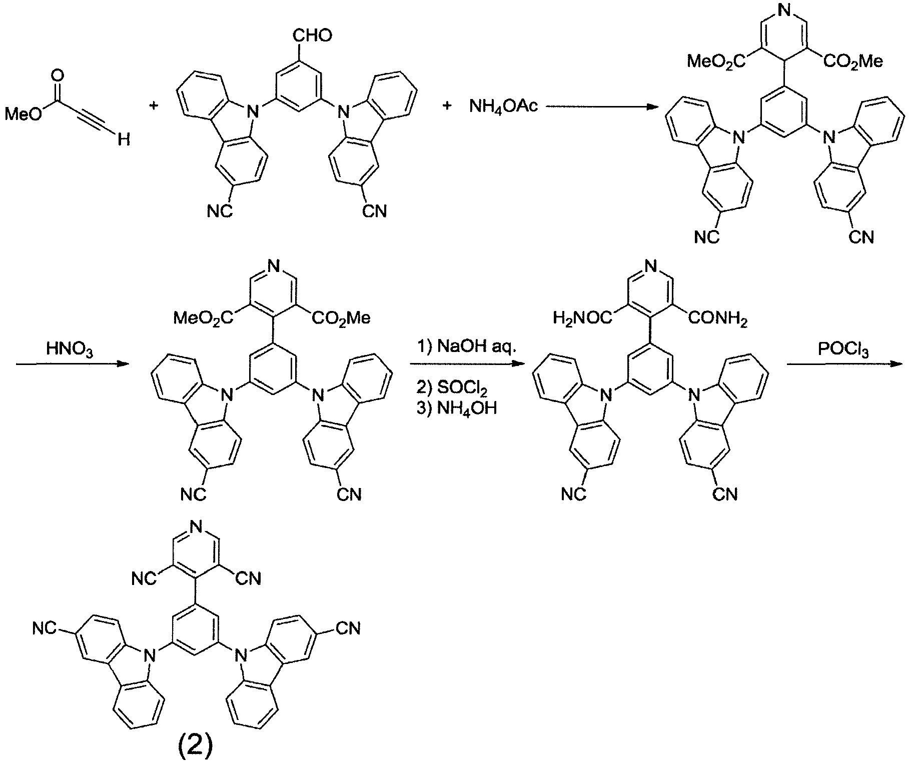

The resulting compound 2 was further subjected to sublimation purification to obtain a sample for evaluation. It was confirmed by ¹H-NMR that the resulting compound was the compound 2. The measurement results of ¹H-NMR were as follows: ¹H-NMR (500 MHz, CDCl₃); δ = 9.24 (s, 2H), 8.47 (s, 2H), 8.18 (d, 2H), 8.10 (t, 1H), 7.93 (d, 2H), 7.77-7.74 (m, 4H), 7.73 (d, 2H), 7.58 (t, 2H), 7.45 (t, 2H).

From the results of analysis of a thin-film by atmospheric photoelectron spectroscopy (AC-3 manufactured by RIKEN KEIKI Co., Ltd.), the HOMO of the compound 2 was estimated to be 6.41 eV. From the long wavelength absorption edge of an absorption spectrum of the compound 1, the HOMO-LUMO band gap was estimated to be 3.57 eV, and the LUMO was estimated to be 2.84 eV.

### [Synthesis Example 3]

In Synthesis Example 3, compound 3 was synthesized in accordance with the following scheme.

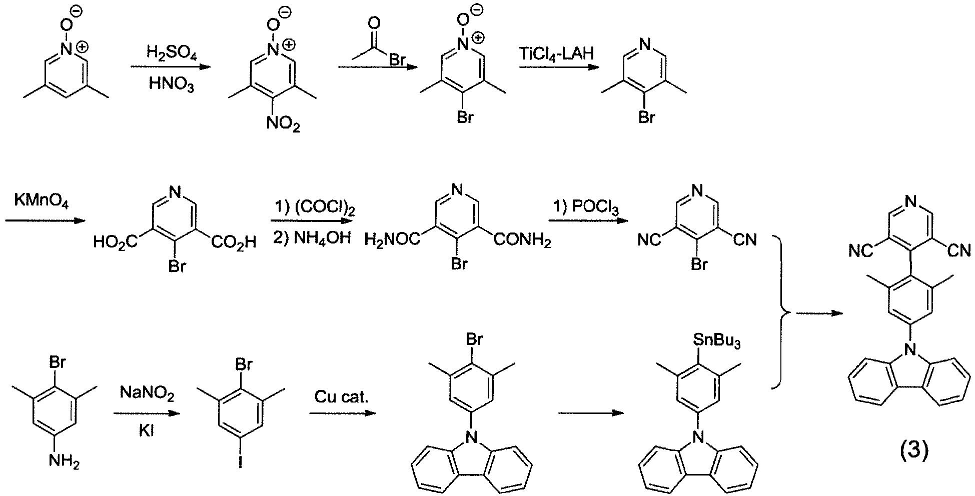

### <Synthesis of 4-Bromopyridine-3,5-Dicarbonitrile>

Fuming nitric acid (35 ml) was added to a sulfuric acid (60 ml) solution containing 3,5-dimethylpyridine N-oxide (25 g), and the mixture was gradually heated and stirred until the internal temperature reached 90°C. Disappearance of the raw materials was confirmed with the reaction monitored by HPLC, the reaction solution was then cooled to room temperature, and poured into ice water. The solution was neutralized with a sodium hydroxide aqueous solution, and the resulting solid was collected by filtration. The desired product in the filtrate was extracted with chloroform, and the solid collected by filtration beforehand was added to the extract, and dissolved. The resulting solution was dried over magnesium sulfate, and concentrated under reduced pressure to obtain 30.7 g of 3,5-dimethyl-4-nitropyridine N-oxide (yield: 90%).

Acetyl bromide (40 ml) was added to an acetic acid (70 ml) solution of 3,5-dimethyl-4-nitropyridine N-oxide (6 g) obtained as described above, and the mixture was stirred under an oil bath heating condition at 80°C for 1 hour. After being cooled to room temperature, the reaction solution was poured into ice water, and neutralized with potassium carbonate. From the solution, the desired product was extracted with chloroform, dried over magnesium sulfate, and then concentrated under reduced pressure to obtain 7 g of 3,5-dimethyl-4-bromopyridine N-oxide (yield: 96%).

Under an Ar atmosphere, a THF (90 ml) solution of titanium tetrachloride (4 ml) was cooled with ice water, and a solution of THF (50 ml) containing lithium aluminum hydride (1 g) was added dropwise. The solution was returned to room temperature, stirred for 15 minutes, and then cooled again with ice water. Thereafter, a THF (50 ml) solution of the 3,5-dimethyl-4-bromopyridine N-oxide (7 g) obtained as described above was added dropwise, and the mixture was returned to room temperature, and then stirred for about 3 hours. The reaction solution was poured into about 15% aqueous ammonia (150 ml), and the resulting solid was filtered off. The filtrate was extracted with ethyl acetate, and the extract was purified by silica gel chromatography to obtain 4.5 g of 3,5-dimethyl-4-bromopyridine (yield: 70%).

An aqueous solution (55 ml) containing the 3,5-dimethyl-4-bromopyridine (3.5 g) obtained as described above and potassium permanganate (7.5 g) was heated and refluxed until the system turned from purple to brown. The reaction solution was temporarily cooled, water (55 ml) and potassium permanganate (7.5 g) were added again, and the mixture was heated and refluxed until the reaction system turned from purple to brown. The resulting solid was filtered off, and the filtrate was concentrated under reduced pressure to about 10 ml. Concentrated hydrochloric acid was added dropwise to the concentrated solution to set the pH of the system to 1. The resulting solid was collected by filtration, and dried under reduced pressure to obtain 2.78 g of 4-bromopyridine-3,5-dicarboxylic acid (yield: 60%).

Under cooling with ice, oxalyl chloride (5.5 ml) was added dropwise to a dichloromethane (30 ml) solution of 4-bromopyridine-3,5-dicarboxylic acid (3.16 g), and a catalytic amount of DMF, and the mixture was heated and refluxed for 3 hours. The reaction solution was returned to room temperature, the solvent was then distilled off under reduced pressure, and the residue was dissolved in 1,4-dioxane (10 ml). The solution was added dropwise to aqueous ammonia, and the resulting solid was collected by filtration, and dried under reduced pressure to obtain 2.14 g of 4-bromopyridine-3,5-dicarboxamide (yield: 68%).

Under cooling with ice, phosphorus oxychloride (1 ml) was added dropwise to a DMF (10 ml) solution of 4-bromopyridine-3,5-dicarboxamide (0.48 g) obtained as described above. The mixture was returned to room temperature, and stirred for 20 hours, and the reaction solution was poured into ice water. The reaction solution was neutralized, the desired product was then extracted with ethyl acetate, and the extract was concentrated to precipitate a solid. The resulting solid was recrystallized using 2-propanol, and the recrystallized product was then collected by filtration, and dried under reduced pressure to obtain 0.2 g of 4-bromopyridine-3,5-dicarbonitrile (yield: 48%).

### <Synthesis of 9-[3,5-Dimethyl-4-(Tributylstannyl) Phenyl]-Carbazole>

Water (20 ml) and concentrated hydrochloric acid (20 ml) were added to 4-bromo-3,5-dimethylaniline (12 g), and the mixture was stirred at 60°C for 30 minutes. Under cooling with ice, an aqueous solution (20 ml) of sodium nitrite (4.83 g) was added dropwise to the mixture, and an aqueous solution (20 ml) of potassium iodide (11 g) was then added dropwise. The reaction solution was stirred at 60°C for 30 minutes, and then further stirred at room temperature for 30 minutes. The desired product in the reaction solution was extracted with chloroform, and the extract was concentrated to precipitate a solid. The resulting solid was purified by column chromatography to obtain 13.21 g of 2-bromo-5-iodo-1,3-dimethylbenzene (yield: 71%).

A DMSO (80 ml) solution containing the 2-bromo-5-iodo-1,3-dimethylbenzene (8.6 g) obtained as described above, carbazole (4.2 g), copper iodide (240 mg), L-proline (290 mg) and potassium carbonate (6.94 g) was heated and stirred at 110°C for 40 hours. The reaction solution returned to room temperature was poured into water, and the desired product was extracted with ethyl acetate. The resulting mixture was purified by column chromatography to obtain 8.2 g of 9-(4-bromo-3,5-dimethylphenyl) -carbazole (yield: 93%).

Under cooling with ice, a THF solution (1.25 ml) of 2 M isopropylmagnesium chloride and a hexane solution (3.1 ml) of 1.6 M n-butyllithium were added dropwise to a CPME (25 ml) solution of the 9- (4-bromo-3,5-dimethylphenyl)-carbazole (2.51 g) obtained as described above, and the mixture was stirred at the same temperature for 1 hour. Thereto was added dropwise tributyltin chloride (2.2 ml), and thereafter the mixture was reacted at room temperature for 2 hours. The reaction solution was poured into saturated aqueous ammonium chloride, and the desired product was extracted with hexane. The mixture was purified by column chromatography to obtain 3.79 g of 9-[3,5-dimethyl-4-(tributylstannyl)phenyl)]-carbazole (yield: 94%).

### <Synthesis of Compound 3>

Under a nitrogen atmosphere, a 1,4-dioxane (5 ml) solution containing 9-[3,5-dimethyl-4-(tributylstannyl)phenyl]-carbazole (0.28 g), 4-bromopyridine-3,5-dicarbonitrile (0.1 g), Pd₂(dba)₃ (9 mg), XPhos (5 mg) and CsF (0.167 g) was heated and stirred at 100°C for 24 hours. The reaction solution returned to room temperature was poured into water, and the desired product was extracted with chloroform. The resulting mixture was purified by column chromatography to obtain 50 mg of a compound (3) (yield: 25%).

The resulting compound 3 was further subjected to sublimation purification to obtain a sample for evaluation. It was confirmed by ¹H-NMR that the resulting compound was the compound 3. The measurement results of ¹H-NMR were as follows: ¹H-NMR (500 MHz, CDCl₃); δ = 9.21(s, 2H), 8.15 (d, 2H), 7.52-7.45 (m, 6H), 7.32 (t, 2H), 2.18 (s, 6H).

From the results of analysis of a thin-film by atmospheric photoelectron spectroscopy (AC-3 manufactured by RIKEN KEIKI Co., Ltd.), the HOMO of the compound 2 was estimated to be 5.80 eV. From the long wavelength absorption edge of an absorption spectrum of the compound 1, the HOMO-LUMO band gap was estimated to be 3.18 eV, and the LUMO was estimated to be 2.62 eV.

### [Synthesis Example 4]

3,6-diphenylcarbazole was used in place of the carbazole in <Synthesis of 9-[3,5-Dimethyl-4-(Tributylstannyl)Phenyl]-Carbazole in Synthesis Example 3. Specifically, a DMSO solution containing 2-bromo-5-iodo-1,3-dimethylbenzene, carbazole, copper iodide, L-proline and potassium carbonate was heated and stirred to obtain 9-(4-bromo-3,5-dimethylphenyl)-3,6-diphenylcarbazole. Except for the above, the same procedure as in Synthesis Example 3 was carried out to synthesize the following compound (4).

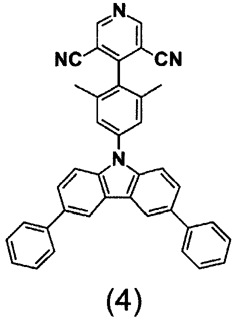

The resulting compound 4 was further subjected to sublimation purification to obtain a sample for evaluation. It was confirmed by ¹H-NMR that the resulting compound was the compound 4. The measurement results of ¹H-NMR were as follows: ¹H-NMR (500 MHz, CDCl₃); δ = 9.23 (s, 2H), 8.41 (d, 2H), 7.76-7.71 (m, 6H), 7.59 (d, 2H), 7.54-7.49 (m, 6H), 7.37 (t, 2H), 2.21(s, 6H). From the result of evaluation by the same procedure as in Synthesis Example 3 above, the compound 4 was estimated to have HOMO of 5.95 eV, HOMO-LUMO band gap of 3.02 eV, and LUMO of 2.93 eV.

### [Evaluation of Light-Emission Characteristics]

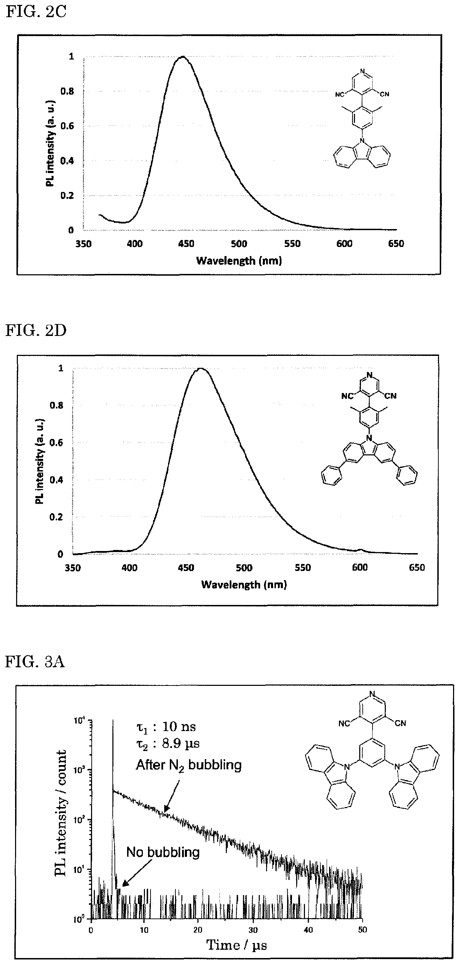

A toluene solution of each of the compounds 1 to 4 was prepared, nitrogen was bubbled into the solution for about 30 minutes, a fluorescence spectrum was then measured at 300 K. The light-emission spectrum of the compound 1 is shown in FIG. 2A, the light-emission spectrum of the compound 2 is shown in FIG. 2B, the light-emission spectrum of the compound 3 is shown in FIG. 2C, and the light-emission spectrum of the compound 4 is shown in FIG. 2D. In FIGS. 2A to 2D, the light-emission intensity is normalized by the value of an intensity at a peak wavelength.

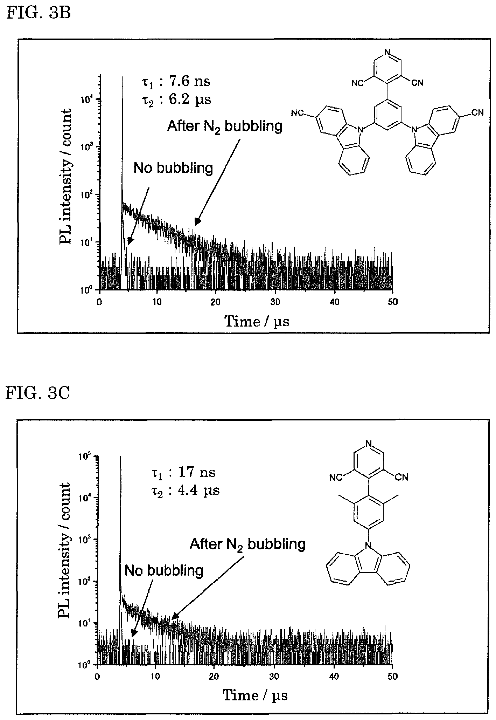

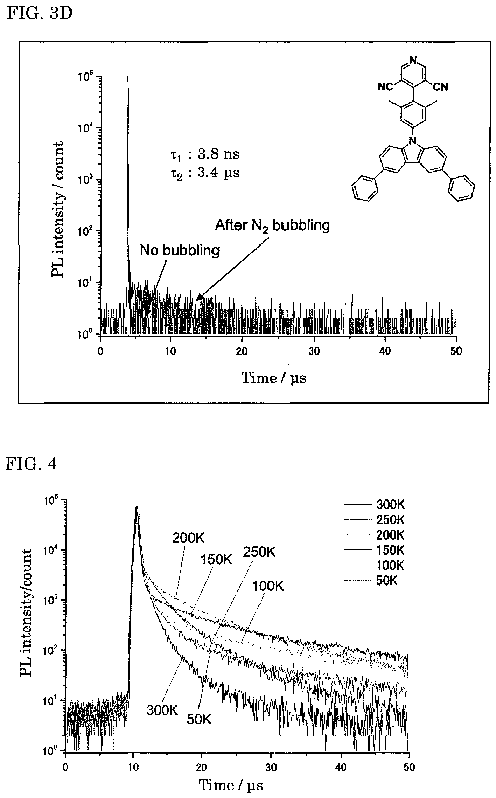

Nitrogen was bubbled into the toluene solution of each of the compounds 1 to 4 for about 30 minutes, and an internal quantum yield was measured using an absolute PL quantum yield measurement apparatus (Quantaurus-QY manufactured by Hamamatsu Photonics K.K.). Further, the transient decay of fluorescence in the toluene solution was measured using a small fluorescence lifetime measuring apparatus (Quantaurus-Tau manufactured by Hamamatsu Photonics K.K.). The fluorescence transient decay curves of the compound 1, compound 2, compound 3 and compound 4 are shown in FIGS. 3A, 3B, 3C and 3D, respectively.

From the obtained transient decay curve, it was determined that a component with a short light-emission lifetime was fluorescent, and a component with a long light-emission lifetime was delayed fluorescence, and the light-emission lifetimes (emission intensity half-lives) τ₁ and τ₂ of the respective components were calculated. In each of the compounds 1 to 4, fluorescence with a lifetime τ₁ in ns order and delayed fluorescence with a lifetime τ₂ in ps order were observed.

It is known that when oxygen exists in the environment, excitons are quenched (energy is transferred from the triplet excited state of the light-emitting material to oxygen molecules), and therefore the lifetime of delayed fluorescence is shortened (see, for example, Endo, A. et al, Appl. Phys. Lett., 98, 83302 (2011)). The transient decay of fluorescence was measured without bubbling nitrogen into the solution, and the result showed that the lifetime τ₂ was shortened in all of the compounds 1 to 4 (see "no bubbling" in FIGS. 3A to 3D). From these results, it was confirmed that the compounds 1 to 4 were materials that emit delayed fluorescence.

The light-emission peak wavelength, the internal quantum yield, the lifetime (τ₁) of the fluorescence component, and the lifetime (τ₂) of the delayed fluorescence component in the toluene solution for each of the compounds 1 to 4 are shown in Table 1.

**Table 1**

| Compound | Properties in toluene solution | | | |
|---|---|---|---|---|
| | Light-emission peak wavelength (nm) | Internal quantum yield (%) | τ₁ (ns) | τ₂ (µs) |
| Compound 1 | 492 | 44.6 | 10.0 | 8.9 |
| Compound 2 | 459 | 10.7 | 7.6 | 6.2 |
| Compound 3 | 445 | 84.4 | 17.0 | 4.4 |
| Compound 4 | 463 | 93.9 | 3.8 | 3.4 |

From the above results, it was confirmed that the organic EL material of the present invention was useful as a light emitting material that emits delayed fluorescence. The compound 1 had a high quantum yield. In the compound 2 having a cyano group as an electron-withdrawing substituent on the carbazole ring, the light-emission peak wavelength was shifted to blue as compared to the compound 1, and thus it was suggested that the compound 2 was useful as a blue delayed fluorescent material. The compound 3 had a shorter light-emission peak wavelength and a higher quantum yield as compared to the compound 2. In the compound 4 having a phenyl group as an electron-donating substituent on the carbazole ring of the compound 3, the light-emission peak was shifted to a longer wavelength side as compared to the compound 3, and the compound 4 had an extremely high quantum yield.

### [Evaluation of Organic Photoluminescence Element Using Compound 4]

### (1) Evaluation with Single Thin-Film

A 100 nm-thick thin-film (light-emitting layer) of the compound 4 was formed on a silicon substrate by a vacuum vapor deposition method to obtain an organic photoluminescence element. A time-resolved spectrum of fluorescence from the thin-film in irradiation with laser light having a wavelength of 290 nm was measured by a streak camera (C 4334 manufactured by Hamamatsu Photonics K.K.) using an absolute quantum yield measurement apparatus (C9920-02 manufactured by Hamamatsu Photonics K.K.) at temperatures of 50 K, 150 K, 200 K, 250 K and 300 K. Transient decay curves of fluorescence are shown in FIG. 4. From the transient decay curves of FIG. 4, it was confirmed that the thin-film of the compound 4 was a thermally activated delayed fluorescence with the delayed fluorescence component changing with an increase in temperature.

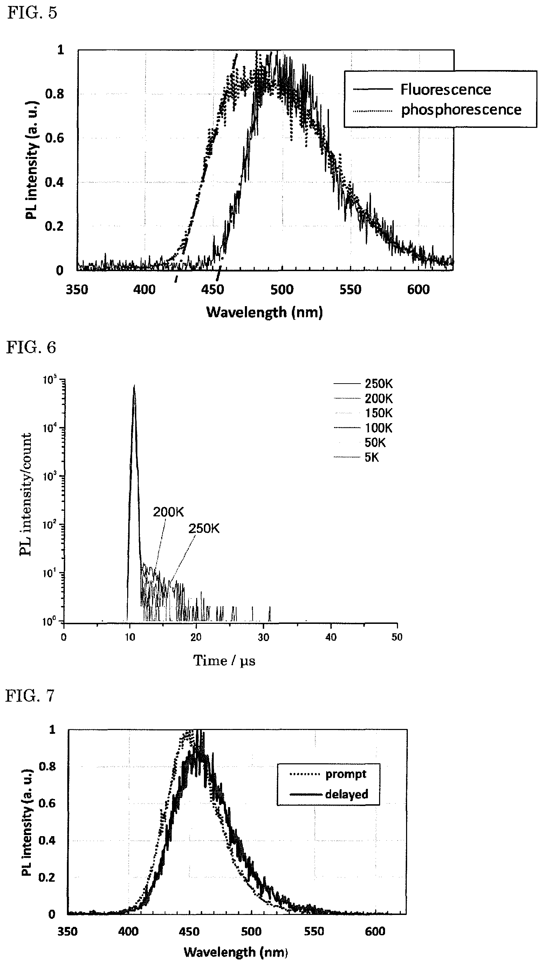

A fluorescence spectrum and a phosphorescence spectrum at a temperature of 300 K over a time of 10 ms are shown in FIG. 5. A tangent line was drawn to the falling edge of the fluorescence spectrum and the phosphorescence spectrum on the short wavelength side in FIG. 5, and the wavelength λedge at the intersection of the tangent line and the horizontal axis was determined. A difference ΔE_{ST} between the lowest excited singlet energy level ES₁ determined from λedge = 455 nm in the fluorescence spectrum on the short wavelength side and the lowest excited triplet energy level ET₁ determined from λedge = 427 nm in the phosphorescent spectrum on the short wavelength side was 0.17 eV.

### (2) Evaluation of Co-Deposited Film with Host Material

On a silicon substrate, mCP as a host material and the compound 4 as a dopant material were co-deposited at a weight ratio of 94 : 6 by a vacuum vapor deposition method, so that a 100 nm-thick thin-film was formed to obtain an organic photoluminescence element. A time-resolved spectrum of fluorescence from the thin-film in irradiation with laser light having a wavelength of 290 nm was measured by a streak camera at temperatures of 5 K, 50 K, 150 K, 200 K, 250 K and 300 K. Transient decay curves are shown in FIG. 6, and Spectra of fluorescence (prompt) and delayed fluorescence (delayed) in 100 ns at a temperature 300 K are shown in FIG. 7. From the transient decay curve in FIG. 6, it was confirmed that the co-deposited film of mCP as a host material and the compound 4 as a dopant material emitted thermally activated delayed fluorescence with the delayed fluorescence component changing with an increase in temperature.

### [Examination of Host Material]

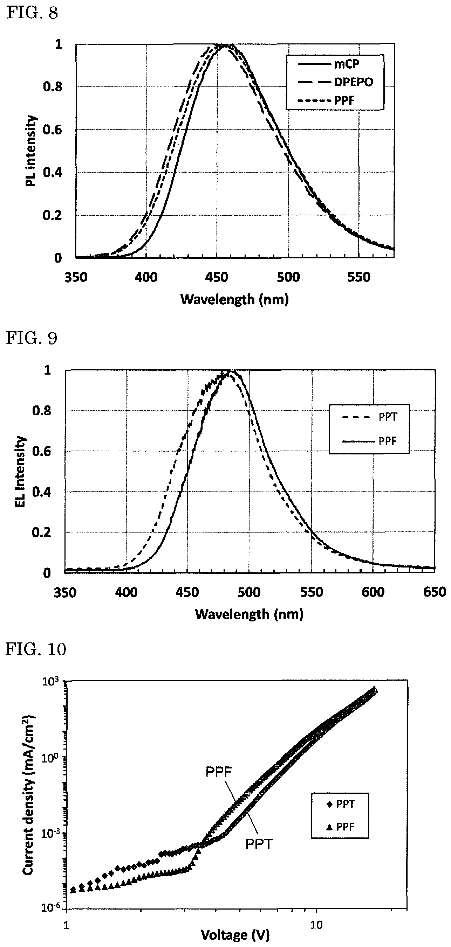

The host material and the compound 4 were co-deposited at a weight ratio of 94 : 6 with each of mCP, DPEPO and PPF used as host materials. A PL internal quantum yield was measured under a nitrogen atmosphere, and a light-emission spectrum and a transient decay of fluorescence in air were measured. For each of the host materials, the internal quantum yield, the emission peak wavelength, and the lifetime (τ₁) of the fluorescence component are shown in Table 2, and the light-emission spectrum is shown in FIG. 8.

**Table2**

| Host material | Internal quantum yield (%) | Light-emission peak wavelength (nm) | τ₁ (ns) |
|---|---|---|---|
| mCP | 79.1 | 460 | 2.90 |
| DPEPO | 96.3 | 450 | 3.00 |
| PPF | 100.0 | 453 | 3.26 |

FIG. 8 and Table 2 show that when the compound 4 as a dopant material is used in combination with a host material, the light-emission wavelength is shifted to blue as compared to a case where the compound 4 is used alone, and therefore it is considered that energy is sufficiently transferred from the host material to the dopant material. In particular, when PPF is used as a host material, the internal quantum yield reaches a theoretical limit of 100%, and thus it is apparent that extremely high luminous efficiency is exhibited.

### [Preparation of Organic EL Element Using Compound 4]

### (1) Element Using PPT Host

On a glass substrate having a patterned ITO electrode (thickness: 50 nm), a bottom emission type evaluation element having a 0.75 mm dot-like light-emitting region was prepared by the following procedure.

Hexaazatriphenylene carbonitrile (HAT-CN) was formed on the ITO electrode by vacuum vapor deposition to form a hole injection layer with a thickness of 5 nm. α-NPD was deposited thereon by vacuum vapor deposition to form a hole transport layer with a thickness of 35 nm.

On the hole transport layer, mCP was deposited in a thickness of 10 nm by vacuum evaporation, and PPT as a host material and the compound 4 as a dopant material were co-deposited thereon at a weight ratio of 90 : 10 to form a light-emitting layer with a thickness of 30 nm. PPT was deposited thereon in a thickness of 10 nm by vacuum vapor deposition.

On the PPT layer, 1,3,5-tris(1-phenyl-1H-benzimidazol-2-yl)benzene (TPBi) was deposited by vacuum vapor deposition to form an electron transport layer with a thickness of 20 nm. 8-hydroxyquinolinolato-lithium (Liq) was deposited thereon in a thickness of 2 nm as an electron injection layer by vacuum vapor deposition, and aluminum was formed thereon in a thickness of 100 nm as a cathode.

The organic EL element was taken out under the atmospheric pressure, a current was fed at a voltage in a range of 0.1 to 17 V, and a current density and an external quantum yield were measured.

### (2) Element Using PPF Host

Except that PPF was used in place of PPT, and the co-deposition ratio of the PPF and the compound 4 in the light-emitting layer was 80 : 20, the same procedure as described above was carried out to prepare an organic EL element, and a current density and an external quantum yield were measured at a voltage in a range of 0.1 to 17 V.

FIG. 9 shows the emission spectra (normalized by the maximum light-emission wavelength) of the organic EL elements at a current density of 100 mA/cm². The light-emission peak wavelength of the organic EL element using PPT as a host material was 477 nm, and the light-emission peak wavelength of the organic EL element using PPF was 486 nm. There was no difference in spectral shape in any of the organic EL elements in a current density range of 1 mA to 100 mA.

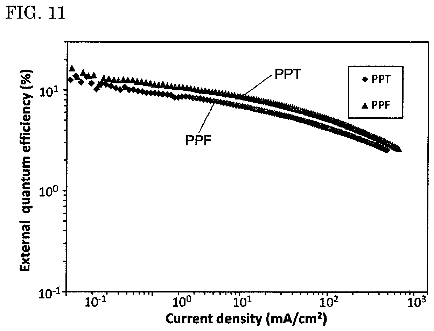

FIG. 10 shows a graph obtained by plotting a relationship between a voltage and a current density, and FIG. 11 shows a graph obtained by plotting a relationship between a current density and an external quantum yield for each organic EL element. Table 3 shows a light-emission peak wavelength and an external quantum yield at each of current densities of 1 mA/cm², 10 mA/cm² and 100 mA/cm² for each element.

**Table3**

| Element | Light-emission peak wavelength (nm) | External quantum yield (%) | | |
|---|---|---|---|---|
| | | 1mA/cm² | 10mA/cm² | 100mA/cm² |
| (1) PPT | 477 | 9.0 | 6.5 | 3.8 |
| (2) PPF | 486 | 11.0 | 8.1 | 4.7 |

As shown in FIG. 10, the organic EL element using the light-emitting material of the present invention exhibited an external quantum yield of about 10% or more at a current density of 1 mA/cm², which exceeds the maximum value (5%) of the external quantum yield of a general fluorescent organic EL element. In addition, even when a voltage of 10 V or more was applied, the current density increased with an increase in voltage.

## Claims

1. An organic EL material comprising a compound represented by the following general formula (I): 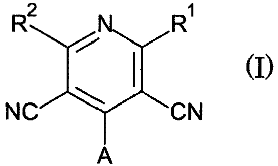 wherein in the general formula (I),
R¹ and R² are each independently a hydrogen atom or any substituent, and
A is a substituent in which at least one heteroaryl group optionally having a substituent, or at least one arylamino group optionally having a substituent is bonded to a carbon atom at the 4-position of the pyridine ring directly or through other aromatic group.

2. The organic EL material according to claim 1, wherein the compound represented by the general formula (I) has an energy difference ΔE_{ST} between Si energy and T₁ energy of 0.3 eV or less.

3. The organic EL material according to claim 1 or 2, wherein R¹ and R² in the general formula (I) are each independently a substituent selected from the group consisting of a hydrogen atom, a cyano group, a halogen-substituted alkyl group, a halogen, a pyridyl group optionally having a substituent, and an electron-donating aromatic group.

4. The organic EL material according to claim 3, wherein each of R¹ and R² in the general formula (I) is a hydrogen atom.

5. The organic EL material according to any one of claims 1 to 4, wherein in the general formula (I), the substituent A bonded to the carbon atom at the 4-position of the pyridine ring is a substituted aryl, and the substituent of the substituted aryl includes a heteroaryl group or an arylamino group optionally having a substituent.

6. The organic EL material according to any one of claims 1 to 5, wherein the substituent A in the general formula (I) includes a heteroaryl group optionally having a substituent.

7. The organic EL material according to claim 6, wherein the heteroaryl group optionally having a substituent includes a nitrogen-containing aromatic heterocyclic ring.

8. The organic EL material according to claim 7, wherein the nitrogen-containing aromatic heterocyclic ring is a carbazole ring.

9. The organic EL material according to claim 8, wherein the substituent A in the general formula (I) is a substituent represented by the following general formula (II): 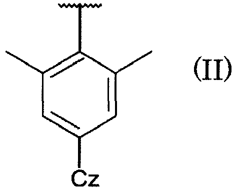 wherein Cz in the general formula (II) is a carbazolyl group optionally having a substituent.

10. The organic EL material according to claim 8, wherein the substituent A in the general formula (I) is a substituent represented by the following general formula (III): 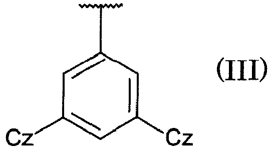 wherein Cz in the general formula (III) is a carbazolyl group optionally having a substituent.

11. The organic EL material according to any one of claims 5 to 8, wherein the substituted aryl further has a substituent different from the heteroaryl group at a position adjacent to a bonding position to the pyridine ring.

12. The organic EL material according to any one of claims 6 to 8, wherein the substituent A in the general formula (I) is a (substituted aryl) amino group with a nitrogen atom bonded to the carbon atom at the 4-position of the pyridine ring, and the substituent of the substituted aryl includes a heteroaryl group optionally having a substituent.

13. The organic EL material according to claim 12, wherein the substituent A in the general formula (I) is a substituent represented by the following general formula (IV): 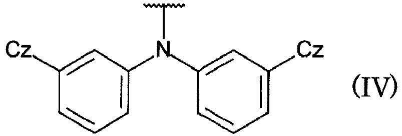 wherein Cz in the general formula (IV) is a carbazolyl group optionally having a substituent.

14. The organic EL material according to any one of claims 1 to 13, wherein the organic EL material is a material that can emit delayed fluorescence.

15. An organic EL element comprising: a pair of electrodes; and a plurality of organic layers including a light-emitting layer disposed between the pair of electrodes, wherein
at least one of the plurality of organic layers contains an organic EL material according to any one of claims 1 to 14.

16. The organic EL element according to claim 15, wherein the compound is contained in the light-emitting layer.

17. The organic EL element according to claim 16, wherein the light-emitting layer contains a dopant material and a host material, and the dopant material is the organic EL material.

18. A lighting fixture including an organic EL element according to any one of claims 13 to 17.

19. A display device including an organic EL element according to any one of claims 13 to 17.
